# EUROPÄISCHE PATENTANMELDUNG

(11) **EP 1 810 669 A1**
(43) Veröffentlichungstag der Anmeldung: **25.07.2007**
(21) Anmeldenummer: 06100616.9
(22) Anmeldetag: 19.01.2006
(51) Int. Cl.: A61K 9/16, A61K 31/485

(54) **Darreichungsform für Buprenorphin**

(71) Anmelder: Hermann, Holger Lars, 8834 Schindellegi (CH)
(72) Erfinder: Hermann, Holger Lars, 8834 Schindellegi (CH)
(74) Vertreter: Hepp, Dieter

(57) **Zusammenfassung**

Die vorliegende Erfindung betrifft ein Pharmazeutische Präparat in Form eines Granulats oder einer Paste, umfassend Buprenorphin, ein mucoadhäsives Additiv und ein in wässrigem Medium quellfähiges Polymer, und dessen Verwendung als Substitutionsmittel zur Behandlung von Drogensucht.

## Beschreibung

Die vorliegende Erfindung betrifft eine neue Darreichungsform für Buprenorphin.

Die Drogensucht ist nach wie vor ein Problem in unserer Gesellschaft. Es ist hinlänglich bekannt, dass die Einnahme bestimmter Substanzen wie beispielsweise Heroin zu einer Abhängigkeit führt. Man unterscheidet hierbei zwischen einer physischen Abhängigkeit und der psychischen Drogenabhängigkeit (Drogensucht). Die physische Drogenabhängigkeit tritt bei einem abrupten Absetzen oder einer signifikanten Verringerung der Menge der eingenommenen Substanz auf. Es kommt zu Entzugserscheinungen wie Erbrechen und Krämpfen des Magen-Darm-Trakts. Hiervon unterscheidet man die eigentliche Drogensucht, die psychische Abhängigkeit, welche als eigene neurologische Erkrankung angesehen wird. Anzeichen von Drogensucht sind die fehlende Kontrolle über die eigene Verwendung der Droge bis hin zur Selbstgefährdung sowie das zwanghafte Verlangen, in den Besitz der Droge zu gelangen.

Drogensucht wird derzeit behandelt, indem man den Patienten substituiert, d.h. von einer gefährlichen auf eine weniger gefährliche Droge umstellt. Aus verschiedenen Gründen findet hierbei aber nicht jede Droge Akzeptanz bei den Patienten. Als Substitutionsmittel wird Methadon, Heroin, Buprenorphin und Morphin eingesetzt.

Buprenorphin ist ein halbsynthetisches Opioid mit der Formel:

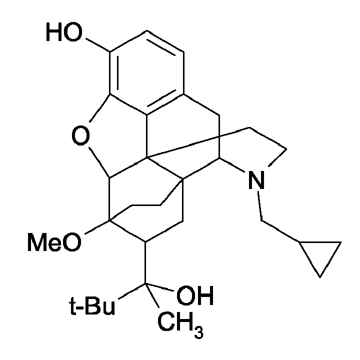

Es ist unter den Handelsnamen Temgesic^{®} und Subutex^{®} erhältlich. Buprenorphin wird als Drogensubstitutionsmittel eingesetzt. Buprenorphin unterscheidet sich von anderen Opiaten dadurch, dass es an den Opiat-Rezeptoren nur partiell agonistisch wirkt. Allerdings weist Buprenorphin eine deutlich höhere Rezeptoraffinität als beispielsweise Morphin auf. Dies führt dazu, dass die Wirkung von Buprenorphin bei einer bestimmten Konzentration ihr Maximum erreicht und nicht mehr verstärkt werden kann (Ceiling-Effekt), nicht einmal durch Einnahme anderer Opiate, da diese das Buprenorphin nicht vom Rezeptor verdrängen können.

Buprenorphin muss zum Erreichen einer gewünschten Wirkung daher genau dosiert werden, führt dann aber zu geringeren Entzugserscheinungen als andere Opiate.

In der Drogensubstitution wird Buprenorphin in Tablettenform abgegeben. Die Tablette muss jedoch sublingual eingenommen werden, d.h. unter der Zunge aufgelöst werden. Wird die Tablette geschluckt, gelangt der Wirkstoff in den Magen-Darm-Trakt, in welchem das Opiat schnell metabolisiert wird und somit eine geringe Bioverfügbarkeit aufweist. Nur bei Aufnahme des Wirkstoffs über die Mundschleimhaut ist eine gewisse Bioverfügbarkeit von Buprenorphin gegeben. Allerdings ist auch hier die Bioverfügbarkeit des Opiats nicht zufrieden stellend: Die Tablette hat eine nicht unbeträchtliche Zerfallszeit, die bei gepressten Tabletten selbst unter günstigen Voraussetzungen mindestens einige Minuten beträgt, bei den im Handel erhältlichen Buprenorphin-Tabletten in der Regel etwa 5 bis 10 Minuten. Während dieser Zeit muss der Patient ein Fremdkörpergefühl im Mund erdulden. Während des Zerfalls hat der Wirkstoff keinen direkten Kontakt mit der Mundschleimhaut, sondern wird in den Speichel freigesetzt. Durch Schlucken des Speichels wird ein nicht unerheblicher Teil des Wirkstoffs unerwünschterweise in den Magen-Darm-Trakt befördert.

Zusammen mit der ohnehin geringeren opioiden Wirkung von Buprenorphin aufgrund der vorstehend beschriebenen Eigenschaft, nur als partieller Agonist zu wirken, führt dies dazu, dass die sublinguale Drogensubstitution mit Buprenorphin bei den Patienten keine besonders hohe Akzeptanz gefunden hat.

Somit steht bei der Drogensubstitution nach wie vor das Methadon im Vordergrund. Allerdings ist die Wirkung von Methadon individuell verschieden und daher nicht einfach zu verabreichen. Methadon macht ebenfalls abhängig. Die Entzugserscheinungen bei Methadon sind sogar länger anhaltend als bei Heroin. Methadon führt wie alle Opiate zu Verstopfung, da es lähmend auf die Magen-Darm-Muskulatur wirkt. Zudem scheint Methadon erhebliche Nebenwirkungen zu besitzen, welche denen des vom Markt genommenen Schmerzmittels Vioxx^{®} entsprechen.

Da die Wirkprofil von Buprenorphin dieses Opiat grundsätzlich zur Drogensubstitution geeignet erscheinen lässt, wäre es wünschenswert, eine Darreichungsform von Buprenorphin bereitzustellen, durch welche die Bioverfügbarkeit von Buprenorphin erhöht wird. Allerdings sollte diese Darreichungsform so beschaffen sein, dass ein Risiko einer missbräuchlichen intravenösen Gabe von Buprenorphin weitgehend ausgeschlossen ist.

In der DE-OS-196 52 188 ist eine Darreichungsform für Buprenorphin beschrieben, bei welcher der Wirkstoff in einer in wässrigen Medien zerfallsfähigen, folien-, papier-, oder oblatenförmigen Darreichungsform enthalten ist. Dadurch wird die Aufnahme des Wirkstoffs durch die Mundschleimhaut erhöht. Allerdings besteht auch bei dieser Darreichungsform ein nicht unerhebliches Risiko einer missbräuchlichen intravenösen Einnahme.

Es bestand daher ein Bedarf nach einer anderen und verbesserten Darreichungsform für Buprenorphin.

Diese Aufgabe wird gemäss der vorliegenden Erfindung gelöst durch ein Pharmazeutische Präparat in Form eines Granulats oder einer Paste, umfassend Buprenorphin, ein mucoadhäsives Additiv und ein in wässrigem Medium quellfähiges Polymer.

Durch die Anwesenheit des mucoadhäsiven Additivs kommt es zu einem Anhaften des Granulats oder der Paste an der Mundschleimhaut. Der Wirkstoff ist somit direkt am Resorptionsort und wird vom Körper direkt über die Mundschleimhaut aufgenommen. Die Wirkstoffaufnahme erfolgt unter Umgehung des Magen-Darm-Trakts. Es wird dadurch eine hohe Bioverfügbarkeit des Buprenorphins erreicht. Es wird eine bessere Dosierung des Burprenorphins möglich, was auch im Sinn einer Verringerung der Toleranzentwicklung erwünscht ist.

Zudem löst sich die erfindungsgemässe Darreichungsform im Mund viel schneller auf als die bisher verwendeten Tabletten. Das vorstehend beschriebene Problem der langen Verweildauer des Präparats im Mund wird signifikant reduziert.

Auf der anderen Seite enthält die erfindungsgemässe Darreichungsform ein in wässrigem Medium quellfähiges Polymer. Neben de positiven Wirkung auf die Freisetzungsgeschwindigkeit des Buprenorphins führt die Anwesenheit dieser Komponente zu einem Schutz vor missbräuchlicher intravenöser Einnahme des erfindungsgemässen Präparats: Bei dem Versuch, das erfindungsgemässe Präparat zur intravenösen Einnahme in einem wässrigen Medium zu lösen, kommt es zu einer irreversiblen Quellung mit einer entsprechenden Volumenzunahme. Als Ergebnis kann das Präparat nicht mehr in einer Spritze aufgezogen werden.

Das Buprenorphin kann gemäß der vorliegenden Erfindung in allen üblichen Darreichungsformen verabreicht werden. Bevorzugt sind physiologisch akzeptable wasserlösliche Salze wie das Buprenorphin-Hydrochlorid.

Beispiele für mucoadhäsive Additive sind Polyacrylsäure, Carboxymethylcellulose, Traganth, Alginsäure, Gelatine, Hydrosymethylcellulose, Methylcellulose und Gummi Arabicum. Darüber hinaus ist von verschiedenen nicht mucoadhäsiven Stoffen bekannt, dass sie in bestimmten Mischungsverhältnissen ebenfalls mucoadhäsive Eigenschaften ausbilden. Ein Beispiel für ein solches Gemisch ist Glycerinmonooleat/Wasser im Verhältnis 84 : 16.

Als in wässrigem Medium quellfähige Polymere eignen sich insbesondere Polymere aus folgender Gruppe: Stärke und Stärkederivate, Dextran; Cellulosederivate, wie Carboxymethylcellulose, Hydroxypropylcellulose, Hydroxyethylcellulose, Hydroxypropylmethylcellulose, Hydroxypropylethylcellulose, Natrium-Carboxymethylcellulose, Ethyl-oderPropylcellulose ; Polyacrylsäure, Polyacrylate, Polyvinylpyrrolidone, Polyethylenoxid-Polymere, Polyacrylamide, Polyethylenglykol, Gelatine, Kollagen, Alginate, Pectine, Pullulan, Traganth, Chitosan, Alginsäure, Arabinogalactan, Galactomannan, Agar- Agar, Agarose, Carrageen, und natürliche Gummen.

Der Anteil an quellfähigem Polymer in dem erfindungsgemässen Präparat beträgt vorzugsweise 5 bis 95 Gew. -%, besonders bevorzugt 15 bis 75 Gew. -%, bezogen auf die Trockenmasse einer Darreichungsform.

In dem erfindungsgemäßen Präparat sind 1 bis 2000 mg, vorzugsweise 100 mg bis 1500 mg Buprenorphin enthalten. Die Menge an Buprenorphin kann entsprechend den Bedürfnissen des Patienten angepasst werden.

Der Anteil an mucoadhäsivem Additiv in dem erfindungsgemässen Präparat beträgt vorzugsweise 5 bis 95 Gew. -%, besonders bevorzugt 15 bis 75 Gew. -%, bezogen auf die Trockenmasse einer Darreichungsform.

Zusätzlich kann das erfindungsgemässe Präparat Zusatzstoffe enthalten, die üblicherweise zur Herstellung von Granulaten oder Pasten herangezogen werden. Dese Zusatzstoffe sind dem Fachmann bekannt und müssen hier nicht weiter erläutert werden.

Dem Fachmann ist die Herstellung von Granulaten und Pasten bekannt. Eine Beschreibung der üblichen Herstellungsverfahren, welche auch im Fall der erfindungsgemässen Darreichungsformen verwendet werden können, erübrigt sich daher.

In den erfindungsgemässen Zubereitungen können neben dem Buprenorphin noch weitere Wirkstoffe enthalten sein, welche ein erwünschtes Zusammenwirken mit Buprenorphin bewirken.

Die erfindungsgemässe Darreichungsform eignet sich hervorragend als Substitutionsmittel zur Behandlung von Drogensucht. Es ist eine effiziente und schnelle Aufnahme des Wirkstoffs durch die Mundschleimhaut gewährleistet. Es ist also nicht wie bei den Sublingualtabletten eine längere Beobachtung der Patienten bei der Einnahme erforderlich. Die Bioverfügbarkeit des Buprenorphins ist bei der erfindungsgemässen Darreichungsform hoch. Es ist eine exakte Dosierung und Einstellung des Patienten möglich, was einer Toleranzentwicklung entgegenwirkt. Auf der anderen Seite ist ein effizienter Schutz gegen eine missbräuchliche intravenöse Einnahme des Präparats gegeben.

## Patentansprüche

1. Pharmazeutische Präparat in Form eines Granulats oder einer Paste, umfassend Buprenorphin, ein mucoadhäsives Additiv und ein in wässrigem Medium quellfähiges Polymer.

2. Präparat nach Anspruch 1, **dadurch gekennzeichnet, dass** das mucoadhäsive Additiv ausgewählt ist aus der Gruppe bestehend aus Polyacrylsäure, Carboxymethylcellulose, Traganth, Alginsäure, Gelatine, Hydrosymethylcellulose, Methylcellulose, Gummi Arabicum, und einem Gemisch aus Glycerinmonooleat/Wasser im Verhältnis 84 : 16.

3. Präparat nach Anspruch 1 oder 2, **dadurch gekennzeichnet, dass** das in wässrigem Medium quellfähige Polymer ausgewählt ist aus der Gruppe bestehend aus Stärke und Stärkederivate, Dextran; Cellulosederivate, wie Carboxymethylcellulose, Hydroxypropylcellulose, Hydroxyethylcellulose, Hydroxypropylmethylcellulose, Hydroxypropylethylcellulose, NatriumCarboxymethylcellulose, Ethyl-oderPropylcellulose ; Polyacrylsäure, Polyacrylate, Polyvinylpyrrolidone, Polyethylenoxid-Polymere, Polyacrylamide, Polyethylenglykol, Gelatine, Kollagen, Alginate, Pectine, Pullulan, Traganth, Chitosan, Alginsäure, Arabinogalactan, Galactomannan, Agar- Agar, Agarose, Carrageen, und natürlichen Gummen.

4. Präparat nach einem der Ansprüche 1 bis 3, **dadurch gekennzeichnet, dass** es Buprenorphin in Form eines physiologisch akzeptablen wasserlöslichen Salzes enthält.

5. Präparat nach einem der Ansprüche 1 bis 4, **dadurch gekennzeichnet, dass** es Buprenorphin in einer Menge von 1 bis 2000 mg, vorzugsweise 100 mg bis 1500 mg, enthält.

6. Präparat nach einem der Ansprüche 1 bis 5, **dadurch gekennzeichnet, dass** der Anteil an in wässrigem Medium quellfähigen Polymer 5 bis 95 Gew. -%, besonders bevorzugt 15 bis 75 Gew. -%, bezogen auf die Trockenmasse einer Darreichungsform beträgt.

7. Präparat nach einem der Ansprüche 1 bis 6, **dadurch gekennzeichnet, dass** der Anteil an mucoadhäsivem Additiv 5 bis 95 Gew. -%, besonders bevorzugt 15 bis 75 Gew. -%, bezogen auf die Trockenmasse einer Darreichungsform beträgt.

8. Verwendung einer Kombination von Buprenorphin, einem mucoadhäsiven Additiv und einem in wässrigem Medium quellfähigens Polymer zur Herstellung eines pharmazeutische Präparats in Form eines Granulats oder einer Paste als Substitutionsmittel zur Behandlung von Drogensucht.
